(19) Europäisches Patentamt
European Patent Office
Office européen des brevets

(11) EP 4 535 098 A1

(12) EUROPEAN PATENT APPLICATION

(43) Date of publication:
09.04.2025 Bulletin 2025/15

(21) Application number: 24204177.0

(22) Date of filing: 02.10.2024

(51) International Patent Classification (IPC):
G05B 13/04 (2006.01)

(52) Cooperative Patent Classification (CPC):
G05B 13/04

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 06.10.2023 JP 2023174352

(71) Applicant: Yokogawa Electric Corporation
Tokyo 180-8750 (JP)

(72) Inventors:
• SAKAKI, Ayumu
  Tokyo, 180-8750 (JP)
• NAMATAME, Tetsushi
  Tokyo, 180-8750 (JP)
• NAKAYA, Makoto
  Tokyo, 180-8750 (JP)

(74) Representative: Hoffmann Eitle
Patent- und Rechtsanwälte PartmbB
Arabellastraße 30
81925 München (DE)

(54) APPARATUS, METHOD, CONTROLLER, CELL CULTURE SYSTEM, AND PROGRAM

(57) Provided is an apparatus, comprising: an acquisition unit that acquires a control variable and a control target of a control object in a process controlled by a controller; a derivation unit that derives a closed-loop transfer function in a vicinity of an operating point of the process based on a control variable model that represents behavior of the control variable in the process and a control function used in control by the controller; and a setting-parameter generation unit that generates a setting parameter for use in control by the controller so that the closed-loop transfer function fits a control target function that is derived from the control target.

FIG.3

**Description**

BACKGROUND

1. TECHNICAL FIELD

The present invention relates to an apparatus, a method, a controller, a cell culture system, and a program.

2. RELATED ART

The patent documents 1 to 12 and the non-patent documents 1 to 2 describe a method or the like "for the provision of optimized process specifications for a cell cultivation process in a reactor system from cultivation data of the cell cultivation process, comprising the steps of: - acquiring cultivation data of the cell cultivation process; and - adapting or generating at least one optimized process specification from acquired cultivation data by applying a Digital Twin obtainable according to the method of any one of claims 1 to 6" (in claim 7 of the patent document 4).

Prior Art Document

Patent Document

Patent Document 1: International Publication No. 2020/252442
Patent Document 2: U.S. Patent Application Publication No. 2019/153381
Patent Document 3: International Publication No. 2020/238918
Patent Document 4: Japanese Translation Publication of a PCT Route Patent Application No. 2022-537799
Patent Document 5: Japanese Patent Application Publication No. 2019-041656
Patent Document 6: International Publication No. 2020/039683
Patent Document 7: International Publication No. 2021/166824
Patent Document 8: Japanese Patent Application Publication No. 2007-202500
Patent Document 9: Japanese Patent Application Publication No. 2018-117567
Patent Document 10: Japanese Translation Publication of a PCT Route Patent Application No. 2009-510606
Patent document 11: Japanese Unexamined Patent Application, Publication No. Sho 59-045872
Patent Document 12: Japanese Patent Application Publication No. 2012-141791

Non-Patent Document

Non-Patent Document 1: Sei Murakami, "Bunkakai 2, Baiyo-koutei renzokuka no genjou to kadai (the current status and challenges of continuous culture process)" Seibutsu Kougakkaishi, Dai 97-kan, Dai 6-gou.
Non-Patent Document 2: Kano, M., Tasaka, K., Ogawa, M., Takinami, A., Takahashi, S., & Yoshii, S. (2011). Extended fictitious reference iterative tuning and its application to chemical processes. Proceedings of the 20114th International Symposium on Advanced Control of Industrial Processes, 379-384

GENERAL DISCLOSURE

In the first aspect of the present invention there is provided an apparatus, comprising: an acquisition unit that acquires a control variable and a control target of a control object in a process controlled by a controller; a derivation unit that derives a closed-loop transfer function in a vicinity of an operating point of the process based on a control variable model that represents behavior of the control variable in the process and a control function used in control by the controller; and a setting-parameter generation unit that generates a setting parameter for use in control by the controller so that the closed-loop transfer function fits a control target function that is derived from the control target.

The above-described apparatus may further comprise: an approximation conversion unit that converts the control variable model into an approximated control variable model in which the control variable model is linearly approximated in a vicinity of an operating point of the process, wherein the derivation unit derives a closed-loop transfer function in the vicinity of the operating point based on the approximated control variable model and the control function. The approximation conversion unit may convert the control variable model representing a viable cell density in the cell culture process into an approximated control variable model.

Any of the above-described apparatuses may further comprise: an adjustment unit that sets, to the control variable model, an amount of feedforward adjustment to offset at least one variable in the process. The adjustment unit may set, to the control variable model, an amount of feedforward adjustment that offsets variables other than a control variable and a manipulated variable.

In any of the above-described apparatuses, the control function may be a function that corresponds to a PID control scheme.

Any of the above-described apparatuses may further comprise: an updating unit that updates the setting parameter based on an operating condition of the process. The updating unit may determine to update the setting parameter if the operating condition of the process exceeds a predetermined threshold or if the change of the operating condition from the time of generation of the setting parameter being used in the control of the process exceeds a predetermined threshold. The updating unit may determine to update the setting parameter according to an updating instruction from an operator.

In any of the above-described apparatuses, the setting-parameter generation unit may generate the setting parameter using a partial model matching method so that the closed-loop transfer function fits the control target function.

Any of the above-described apparatuses may further comprise: a control-target-function setting unit that sets the control target function based on the control target.

In the above-described apparatus, the control-target-function setting unit may set the control target function in a binomial coefficient standard form, a Butterworth standard form, or an ITAE (Integral of Time multiplied by Absolute Error) least standard form.

In the second aspect of the present invention, there is provided a method for controlling, by a controller, at least one of process values of a cell culture process using a setting parameter generated by any of the above-described apparatuses.

In the third aspect of the present invention, there is provided a controller that performs feedback control on at least one of process values of a cell culture process by a setting parameter generated by any of the above-described apparatuses.

In the fourth aspect of the present invention, there is provided a cell culture system, comprising: a culture tank for culturing a cell or a microorganism in a cell culture process; a sensor that measures a process value managed in the cell culture process; an actuator that performs an operation to the culture tank; and the above-described controller that controls the actuator according to a measurement result of the sensor.

In the fifth aspect of the present invention, there is provided a method, comprising: acquiring a control variable and a control target of a control object in a process controlled by a controller; deriving a closed-loop transfer function in a vicinity of an operating point of the process based on a control variable model that represents behavior of the control variable in the process and a control function used in control by the controller; and generating a setting parameter for use in control by the controller so that the closed-loop transfer function fits a control target function that is derived from the control target.

In the sixth aspect of the present invention, there is provided a program that is executed by a computer and causes the computer to function as an acquisition unit that acquires a control variable and a control target of a control object in a process controlled by a controller; a derivation unit that derives a closed-loop transfer function in a vicinity of an operating point of the process based on a control variable model that represents behavior of the control variable in the process and a control function used in control by the controller; and a setting-parameter generation unit that generates a setting parameter for use in control by the controller so that the closed-loop transfer function fits a control target function that is derived from the control target.

Note that the summary clause does not necessarily describe all necessary features of the embodiments of the present invention. The present invention may also be a sub-combination of the features described above.

BRIEF DESCRIPTION OF THE DRAWINGS

Fig. 1 shows a cell culture system 1 according to an embodiment.
Fig. 2 shows a control loop 15 of the cell culture system 1.
Fig. 3 shows a setting apparatus 14 according to an embodiment.
Fig. 4 shows an operation of the setting apparatus 14.
Fig. 5 shows an example of a computer 2200 in which a plurality of aspects of the present invention may be embodied in whole or in part.

DESCRIPTION OF EXEMPLARY EMBODIMENTS

Hereinafter, embodiments of the present invention will be explained. However, the following embodiments do not limit the invention according to the claims. In addition, not all of the combinations of features described in the embodiments are essential to the solution of the invention.

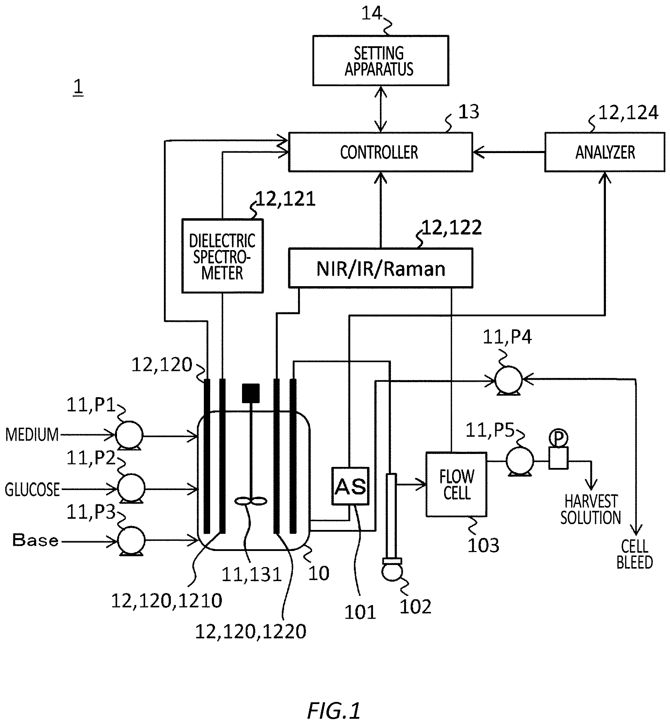

Fig. 1 shows a cell culture system 1 according to the present embodiment. The cell culture system 1 performs culturing to produce a target substance by a biological reaction. The cell culture system 1 may produce, for example, biopharmaceuticals such as antibody drugs, or may culture animal cells such as CHO cells. In addition, the cell culture system 1 may culture cells of vertebrates other than CHO cells, cells of shellfish, insects, or the like, human cells, plant cells, or microbial cells, or alternatively may perform yeast fermentation.

The cell culture system 1 may include a culture tank 10, one or more actuators 11, one or more sensors 12, a controller 13, and a setting apparatus 14. The culture tank 10, one or more sensors 12, the controller 13, and one or more actuators 11 may form one or more control loops (as an example, a closed control loop or an open control loop), and the cell culture system 1 may control the actuator 11 via the controller 13 according to measurement results by the sensors 12.

The culture tank 10 is a container that accommodates a medium and cells to culture the cells or microorganisms in a culture process. In order to promote cell growth, the temperature, the pH, the amount of dissolved oxygen (DO), and the like of the culture liquid in the culture tank 10 may be appropriately managed by the actuator 11. As an example, a perfusion culture may be performed in the culture tank 10, and the medium may be continuously supplied to the culture tank 10 at a stage when the cells have grown to a certain extent (for example, on a second day to a third day from a start of the culture), for an amount of cell removal solution (a harvest solution) which is equal to the supplied amount, to be recovered from the culture tank 10. In this manner, a volume of the culture liquid in the culture tank 10 may be kept at a certain level.

Note that, the culture tank 10 may be provided with a manually operated sampling apparatus 101 or a feed apparatus (not shown). The sampling apparatus 101 may sample the culture liquid in the culture tank 10, and the feed apparatus may administer a feed agent to the culture tank 10 to supplement a source of nutrients. The sampling apparatus 101 and the feed apparatus may be the actuators 11 which are automatically operated.

Each actuator 11 is a driving apparatus that moves physically and performs operations to the culture tank 10. Each actuator 11 may control at least one of the dissolved oxygen concentration, the pH, the temperature, the substrate concentration (glucose concentration), the cell density, the amount of culture liquid, the stirring rate, the pressure, the weight, the volume, the flow rate, or the like of the culture liquid in the culture tank 10. Each actuator 11 may be any of a valve, a pump, a heater, a fan, a motor, and a switch.

In the present embodiment, as an example, the cell culture system 1 may have, as the actuator 11, a pump P1 which supplies a medium to the culture tank 10, a pump P2 which supplies glucose to the culture tank 10, a pump P3 which supplies an alkali (Base) to the culture tank 10, a stirring apparatus 131 which stirs the culture liquid in the culture tank 10, a pump P4 which discharges the cell along with the culture liquid from the culture tank 10, and a pump P5 which discharges a harvest solution from the culture tank 10.

Among these actuators 11, the pump P5 may discharge the harvest solution in the culture tank 10 via a cell removal apparatus 102 and a flow cell 103. The cell removal apparatus 102 may have a filtering action called a TFF (tangential flow filtration) or an ATF (alternating tangential flow filtration) and may discharge the harvest solution from the culture tank 10 while maintaining the cells in the culture tank 10.

Each sensor 12 measures a state of the culture tank 10. Each sensor 12 may measure process values to be managed in a cell culture process. Each sensor 12 may measure a process value (also referred to as a process parameter, PV (Process Value)) indicating the dissolved oxygen concentration, the pH, the temperature, the substrate concentration (glucose concentration), the cell density, the amount of culture liquid, the stirring rate, the pressure, the weight, the volume, the flow rate, or the like, in the culture liquid.

In the present embodiment, as an example, the cell culture system 1 may have, as the sensor 12, a sensor (not shown) which measures the weight of the culture tank 10, a sensor (not shown) which measures the volume and the weight of a fluid that is supplied by the pumps P1 to P3, a sensor (not shown) which measures the density of cells, a dielectric spectrometer 121 which measures a dielectric constant of the culture liquid; a near-infrared/infrared/Raman spectrometer 122 which measures nutritional and metabolic components in the culture liquid; an analyzer 124 which analyzes components of the culture liquid; and an in-line sensor 120 which is immersed in the culture liquid to measure a state of the culture liquid.

Among these sensors 12, the dielectric spectrometer 121 may measure the dielectric constant of the culture liquid according to a current flowing between electrodes 1210 inserted into the culture liquid, and may further measure, from the measured dielectric constant, the number and a viability of the cells or the like in the culture liquid.

The near-infrared/infrared/Raman spectrometer 122 may irradiate the culture liquid with various types of measurement light (near-infrared light, infrared light, monochromatic light) via a light receiving and emitting sensor 1220 inserted into the culture liquid, to measure a near-infrared spectrum, an infrared spectrum, a Raman spectrum, or the like of the light transmitted through or reflected from the culture liquid; and may further measure, from the measured spectrum, glucose, lactate, ammonia, various types of amino acids, or the like in the culture liquid. Note that, in the measurement by the near-infrared/infrared/Raman spectrometer 122, the measurement may be performed by: using a solution in which a component concentration has been adjusted in advance, to construct a calibration model; and converting the spectrum acquired from the light receiving and emitting sensor 1220 immersed in the culture tank 10, into the component concentration by the calibration model. The light receiving and emitting sensor 1220 may be provided in the flow cell 103.

The analyzer 124 may be, for example, a high performance liquid chromatography (HPLC) apparatus, a cell culture analyzer, or the like. The analyzer 124 may analyze the component of the culture liquid sampled from the culture tank 10 by the sampling apparatus 101 at a reference interval (24 hours, as an example), offline, that is, away from the culture tank 10.

The in-line sensor 120 may be immersed in the culture liquid to measure the state of the culture liquid during the execution of the cell culture. As an example, the in-line sensor 120 may measure at least one of fundamental physical and chemical parameters such as the pH, the amount of dissolved oxygen (DO), the temperature, the partial pressure of a gas (partial pressure of oxygen, partial pressure of carbon dioxide, or the like), the osmotic pressure, the nutrient component, the metabolic component, or the target product concentration, in the culture liquid. The electrode 1210 of the dielectric spectrometer 121 and the light receiving and emitting sensor 1220 of the near-infrared/infrared/Raman spectrometer 122 described above may be the in-line sensors 120. Note that, a drift may occur in the measurement result by the in-line sensor 120 due to a change in a culture environment. An example factor that causes the change in the culture environment includes a variation in a mechanical stress that occurs in the culture liquid due to stirring or gas aeration, depletions of a nutrient and oxygen in the culture liquid, an accumulation of a waste product (also referred to as debris, an impurity) such as lactate and ammonia that are produced by the cell.

Among one or more sensors 12 included in the cell culture system 1, at least one sensor 12 may be provided with a denoise filter which removes noise from the measurement result. The denoise filter may cause a delay between when the variation occurs in a measurement target of the sensor 12, and when the variation occurs in the measurement result by the sensor 12.

Each sensor 12 may perform the measurement at a preset cycle. Each sensor 12 may supply process values as measurement results to the controller 13. Each sensor 12 may undergo calibration at an arbitrary timing.

Note that, the process value may be an analytical value associated with at least one of a culture liquid component, a culture environment, or a biomass state that are acquired by sampling the culture liquid at a reference frequency (as an example, once per day) and analyzing it by the analyzer 124. Here, the analytical value associated with the culture liquid component may be a value indicating a component such as glucose, glutamine, glutamic acid, lactate, ammonium ion, or other components, metabolites, or the like that affect cell growth or a quality and production quantity of a target product. The analytical value associated with the culture environment may be the temperature, the pH, the dissolved oxygen concentration, the dissolved carbon dioxide concentration, the osmotic pressure, the amount of culture liquid, the perfusion rate, or the like of the culture liquid. The analytical value associated with the biomass state may be the bacterial cell concentration (viable cell density, total cell density), the growth rate, the consumption rate of nutrient components, such as glucose, of microorganisms, the generation rate of the target product or a by-product, or the cell survival rate or the like. The cell density measured by the dielectric spectrometer 121 or the like may be calculated by analyzing an image captured by staining the culture liquid. The nutrient components and the metabolic components measured by the near-infrared/infrared/Raman spectrometer 122 or the like may be measured using an enzyme sensor or the like.

The controller 13 may control one or more actuators 11 according to the measurement results of one or more sensors 12. For example, the controller 13 may control the actuator 11 by supplying the actuator 11 with a control signal indicating a manipulated variable that is calculated according to the measurement result by the sensor 12. The controller 13 may scale (normalize, as an example) the measurement results of one or more sensors 12 and then use the scaled measurement result to calculate the manipulated variable.

The controller 13 may control the cell culture process in the cell culture system 1 by controlling one or more actuators 11 according to the measurement results of one or more sensors 12. For example, the controller 13 may monitor the state of the culture liquid in the culture tank 10 and perform various types of control (operations of various types of pumps, motors, or the like, temperature controls, and so on) related to the perfusion culture process to control an amount of supply, an amount of recovery, an amount of ventilation, a bleed flow rate, perfusion rate, or the like in the culture tank 10. As an example, the controller 13 may add the nutrient component contained in the culture medium, or an enhancer that enhances the growth rate or the production of the cell, while controlling fundamental process values such as the dissolved oxygen concentration, the pH, the temperature, the substrate concentration (the glucose concentration), the cell density, the amount of culture liquid, and the stirring rate in the culture liquid.

A target value (also referred to as a SV (set value)) of the process value may be set in the controller 13. When the target value is set, the controller 13 may calculate a manipulated variable to bring the process value to the target value (or to be within a reference range of the target value) and control the actuator 11. When the cell culture system 1 has a plurality of control loops 15, the target value may be set in the controller 13 for each control loop 15. The target value of the process value may be set by an operator so that any control target is accomplished.

A setting parameter may be set in the controller 13. The controller 13 uses the setting parameter to perform feedback control on at least one of the process values of the cell culture process. The setting parameter may be a parameter to indicate a control condition for the actuator 11 and may be a parameter, for example, for the feedback control. As an example, the setting parameter for the feedback control may be at least one of a proportional gain, an integral time, or a derivative time. The setting parameter may be a parameter (also referred to as a frequency parameter) indicating an output frequency or a control cycle of a control signal to the actuator 11, or may be an upper/lower limit value of the manipulated variable specified to the actuator 11 (also referred to as the manipulated variable to the actuator 11). The upper/lower limit value of the manipulated variable may be set, as an example, at a rate of 0 to 100 (%), -50 to 50 (%), or the like.

In response to the cell density in the culture liquid reaching the reference value, the controller 13 may keep the cell density in the culture tank 10 within an appropriate range by bleeding operation to discharge the cells along with the culture liquid from the culture tank 10 using the pump P4. The controller 13 may include, as an user interface, a setting screen for the target value of the process value, a trend display function, a data storage function, an output function, and the like.

The setting apparatus 14 is an example of the apparatus and generates a setting parameter for the controller 13 in controlling the cell culture system 1. The setting apparatus 14 may generate a setting parameter according to information related to the control of the cell culture system 1 and set the setting parameter to the controller 13.

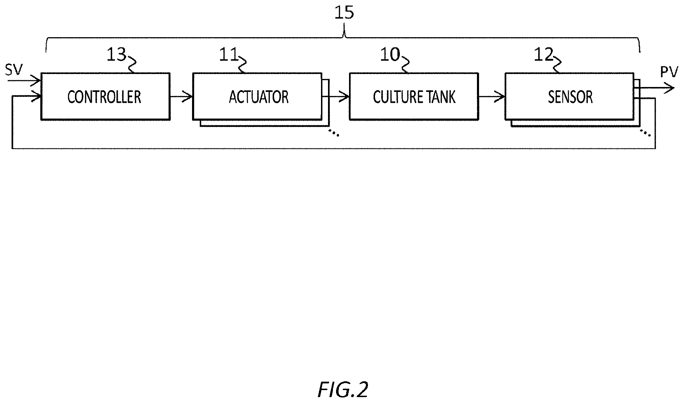

Fig. 2 shows the control loop 15 of the cell culture system 1. The controller 13, the actuator 11, the culture tank 10, and the sensor 12 of the cell culture system 1 may form one or more closed control loops 15.

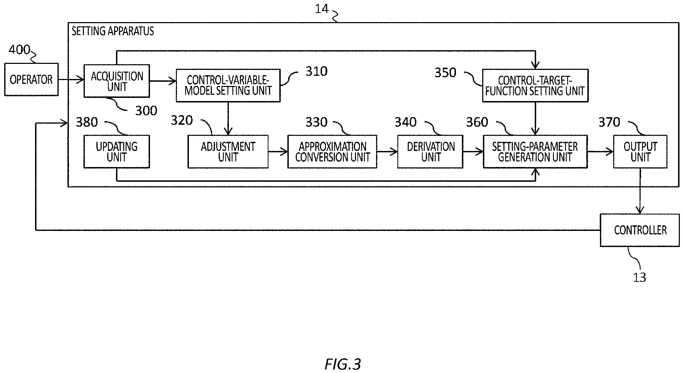

Fig. 3 shows a configuration example of the setting apparatus 14 according to the present embodiment. The setting apparatus 14 includes an acquisition unit 300, a control-variable-model setting unit 310, an adjustment unit 320, an approximation conversion unit 330, a derivation unit 340, a control-target-function setting unit 350, a setting-parameter generation unit 360, an output unit 370 and an updating unit 380.

The acquisition unit 300 may acquire information related to a setting parameter of the controller 13 from an operator 400 and the controller 13. The acquisition unit 300 acquires a control variable and a control target of a control object in the cell culture process controlled by the controller 13. The acquisition unit 300 may acquire one or more control targets and one or more control variables. The acquisition unit 300 may acquire, as the control variable, information to specify a process value (as an example, a type of the process value and a target value, etc.) such as the dissolved oxygen concentration in the culture liquid, the pH, the temperature, the substrate concentration, the cell density, the amount of culture liquid, the stirring rate, the pressure, the weight, the volume, or the flow rate.

Here, the control target acquired by the acquisition unit 300 may be a control performance that should be accomplished during the cell culture process (for example, a target-value-following characteristic, a disturbance-suppression characteristic, or the like), or alternatively may be a target value of the control performance or the like. The control target may be a target associated with a steady-state characteristic, and as an example, may be that the process value when the culture tank 10 is in a steady state is within a reference range of the target value. The control target may be a target associated with a transient characteristic, and as an example, may be that a settling time (also referred to as a response delay time) until the process value reaches the target value (or within the reference range of the target value) is less than a reference time. The control target may be a target associated with the followability to the target value, and as an example, may be that, when a target value of any of the process values is changed for performing the cell culture, a rate of the process value (also referred to as a follow-up rate) to reach the target value (or within the reference range of the target value) exceeds a reference rate.

The control-variable-model setting unit 310 is connected to the acquisition unit 300. The control-variable-model setting unit 310 sets a control variable model that represents behavior of a control variable in the cell culture process. The control-variable-model setting unit 310 may set a control variable model that corresponds to the control variable acquired by the acquisition unit 300.

The adjustment unit 320 is connected to the control-variable-model setting unit 310. The adjustment unit 320 sets, to the control variable model, an amount of feedforward adjustment that offsets at least one variable in the cell culture process.

The approximation conversion unit 330 is connected to the adjustment unit 320. The approximation conversion unit 330 converts the control variable model into an approximated control variable model in which the control variable model is linearly approximated in the vicinity of an operating point of the cell culture process.

The derivation unit 340 is connected to the approximation conversion unit 330. The derivation unit 340 derives a closed-loop transfer function in the vicinity of the operating point based on the control variable model and a control function that is used in control by the controller 13.

The control-target-function setting unit 350 is connected to the acquisition unit 300. The control-target-function setting unit 350 sets a control target function based on the control target. The control-target-function setting unit 350 may set a control target function that satisfies the control target acquired by the acquisition unit 300.

The setting-parameter generation unit 360 is connected to the derivation unit 340 and the control-target-function setting unit 350. The setting-parameter generation unit 360 generates a setting parameter for use in control of the controller 13 so that the closed-loop transfer function fits the control target function derived from the control target. The setting-parameter generation unit 360 may generate the setting parameter using a partial model matching method so that the closed-loop transfer function fits the control target function.

The output unit 370 is connected to the controller 13 and outputs the setting parameter generated to the controller 13.

The updating unit 380 is connected to the setting-parameter generation unit 360. The updating unit 380 updates the setting parameter based on an operating condition of the cell culture process. The updating unit 380 may update the setting parameter according to the operating condition of the cell culture process received from the controller 13.

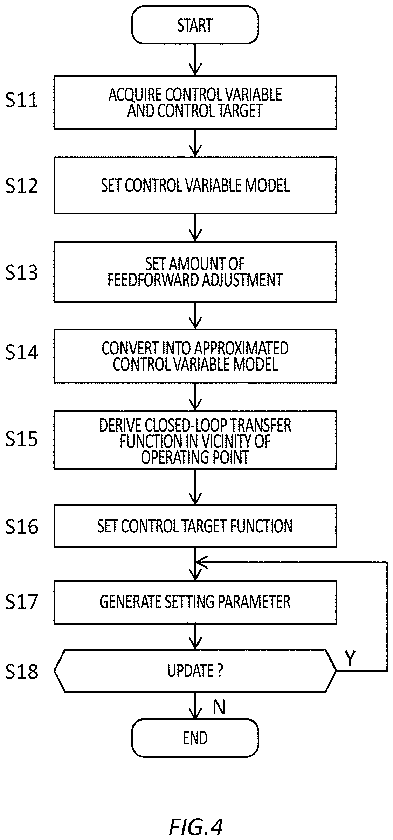

Fig. 4 shows a flow of the setting parameter generation in the setting apparatus 14. The setting apparatus 14 performs processing of steps S11 to S18 to determine a setting parameter by which a desired control target is accomplished.

In step S11, the acquisition unit 300 acquires a control variable and a control target. The acquisition unit 300 may acquire a combination of the control variable and the control target that are input by an operator 400 through a keyboard, a mouse, a touchpad, or the like. The acquisition unit 300 may acquire a combination of one or more control variables and one or more control targets. The acquisition unit 300 may store combinations of the control variables and the control targets and may acquire a combination of the control variable and the control target according to the information (for example, a type of culture tank 10, a type of cells cultured in the culture tank 10, or the like) related to the cell culture process input by the operator 400. Thus, the operator 400 can set, to the setting apparatus 14, the control variable and the control target for the control variable.

In step S12, the control-variable-model setting unit 310 may set a control variable model (as an example, a non-linear differential equation) that represents behavior, in the cell culture process, of the control variable acquired by the acquisition unit 300. The control-variable-model setting unit 310 may set the control variable model according to an operating condition (such as a type of process value, a target value) or the like of the cell culture process acquired through the acquisition unit 300. The control-variable-model setting unit 310 may set a control variable model that represents a relation between the control variable and a process value (such as a manipulated variable) other than the control variable. The control-variable-model setting unit 310 may acquire a control variable model from the operator 400 through the acquisition unit 300 and set the acquired control variable model. The control-variable-model setting unit 310 may prestore one or more control variable models in association with respective control variables, and may set a control variable model, among the stored control variable models, that corresponds to the control variable acquired by the acquisition unit 300.

As an example, a control variable model that is set by the control-variable-model setting unit 310 in the control of the viable cell density in perfusion culture is shown. In the present example, the acquisition unit 300 acquires the viable cell density as a control variable, and the control-variable-model setting unit 310 sets a control variable model of the viable cell density. Behavior of cell growth in the culture tank 10 is described by an exponential temporal change. In addition, in order to maintain a constant culture environment for a long time, the perfusion culture is operated to manage the viable cell density at a certain level, and a bleeding method is used as an operational measure to discharge a part of the cell culture liquid in the culture tank 10 to the outside of the system. Accordingly, the control-variable-model setting unit 310 can define the control variable model of the viable cell density in the cell culture process of the perfusion culture as in Expression 1.

$$\frac{d(V[X_v])}{dt} = \mu V[X_v] - (q_B + q_{Samp})[X_v] \tag{1}$$

$$\frac{dV}{dt} = q_{in} - q_{out} \tag{2}$$

Here, in Expression 1, $[X_v]$ represents the viable cell density, V represents the amount of culture liquid, $\mu$ represents a specific growth rate (a model parameter representing the cell growth per unit cell, per unit time), $q_B$ represents a bleed flow rate (a flow rate of discharge of the cell culture liquid), and $q_{Samp}$ represents a flow rate of discharge to sample the culture liquid for analysis. In Expression 1, for the specific growth rate $\mu$, a representative value may be set which is determined based on an a priori knowledge of the cell line used, such as data of a seed train (passage culturing in a scale-up process). In addition, in Expression 2, $q_{in}$ represents a total flow rate on an inflow side connected to the culture tank 10 and $q_{out}$ represents a total flow rate on an outflow side connected to the culture tank 10. The control-variable-model setting unit 310 may combine Expression 1 and Expression 2 to set the control variable model of the viable cell density as shown in Expression 3.

$$\frac{d[X_v]}{dt} = \mu[X_v] - \frac{1}{V}(q_B - q')[X_v] \tag{3}$$

In Expression 3, q' represents a flow rate of a pump, other than the bleed pump, affecting the viable cell density, and represents an influence of the temporal change of the amount of culture liquid (Expression 2) over the temporal change of the viable cell density.

Note that, the control-variable-model setting unit 310 may set other control variable models of the viable cell density or may, alternatively, set control variable models of other control variables. In order to turn the control loop 15 of the cell culture system 1 into a control algorithm based on a mathematical model of the control object, the control-variable-model setting unit 310 may set the control variable model in a descriptive form of expression, such as a first-principles model according to physical phenomena. In addition, the control-variable-model setting unit 310 may set a black box model according to a statistical approach as the control variable model. Note that, in terms of adjustment of the setting parameter, a preferable model parameter of the control variable model is one whose representative value is known. In addition, the control-variable-model setting unit 310 can grasp a dead time element from sensor characteristics or the like and may set it in the control variable model if its influence on a response delay of the cell culture process is non-negligible. Note that, the control-variable-model setting unit 310 may not set the dead time element if the dead time is unknown, because the response delay (such as a rising time or a settling time) is often more dominant than the dead time in the cell culture process involving biological responses (such as cell growth or substrate consumption).

In step S13, the adjustment unit 320 may set, to the control variable model, an amount of feedforward adjustment that offsets at least one variable other than the control variable acquired by the acquisition unit 300. The adjustment unit 320 may set the amount of feedforward adjustment to the feedforward control performed by the controller 13 in the cell culture process. If interference terms of other control loops or other known external inputs are included in the control variable model, the adjustment unit 320 may set the amount of feedforward adjustment to be used in a disturbance feedforward control. Note that, the disturbance feedforward control is a control to proactively cancel the influence of the disturbance before an output of the control system is disturbed due to the known external actions (for example, the influence of the inference, or the like). The adjustment unit 320 may set the amount of feedforward adjustment, particularly when the controller 13 is an SISO (single input single output) type controller, such as PID (Proportional-Integral-Derivative) controller. The adjustment unit 320 may set the amount of feedforward adjustment that cancels the terms other than the control variable and the manipulated variable, thus making adjustments of the setting parameter in the following steps efficient.

Here, the manipulated variable may be, for example, the amount of supply of the feed medium, the amount of recovery, the bleed flow rate (the discharge flow rate of the culture liquid), the amount of ventilation, the perfusion rate, or the like in the culture tank 10 controlled by the controller 13.

As an example, the adjustment unit 320 may set the amount of feedforward adjustment to the control variable model of the viable cell density as set in Expression 3. The control variable model of Expression 3 includes the interference term related to the flow rate q' of a pump other than the bleed pump, which affects the cell culture process through the discharge flow rate (the harvest flow rate) of the cell removal solution or the charge flow rate of the perfusion medium for the purpose of product recovery and liquid amount management in the cell culture process of the perfusion culture. Accordingly, these flow rates are regarded as a disturbance in the viable cell density control system. Meanwhile, because these flow rates are manipulated variables (manipulation items) to be controlled in the cell culture process, they are also known process values that can be grasped and operated by the operator 400. Thus, the temporal change of the viable cell density due to the variation of these flow rates can be offset by the disturbance feedforward control. The adjustment unit 320 can generate Expression 4 by setting, to the control variable model, the amount of feedforward adjustment according to these flow rates.

$$\frac{d[X_v]}{dt} = \mu[X_v] - \frac{q_{B,FF}}{V}[X_v] \tag{4}$$

$$q_B = q_{B,FF} + q_{B,FB} \tag{5}$$

$$q_{B,FF} = q' \tag{6}$$

In Expression 6, $q_{B,FF}$ represents the feedforward adjustment term (the amount of feedforward adjustment) of the bleed flow rate and $q_{B,FB}$ represents a feedback adjustment term (the amount of feedback adjustment) of the bleed flow rate.

In step S14, the approximation conversion unit 330 converts the control variable model into an approximated control variable model in which the control variable model is linearly approximated in the vicinity of an operating point of the cell culture process. The approximation conversion unit 330 may convert the control variable model into the approximated control variable model by performing the linear approximation on a non-linear term related to the control variable or the manipulated variable of the control variable model in a predetermined range (for example, in a range of the target value $\pm$ $\alpha$) with reference to the target value of at least one process value in the cell culture process. If the control variable model includes a non-linearity, the approximation conversion unit 330 focuses on the vicinity of the operating point and locally converts the dynamic characteristics of the non-linear and complicated feedback loop in a linearly approximating manner, allowing easier processing of the control variable model in the following steps. That is, step S14 is performed to apply a model-based design method of the control system (the partial model matching method) to the control object represented by the non-linear control variable model.

As an example, the approximation conversion unit 330 approximates the control variable model in which the amount of feedforward adjustment is set as in Expression 4, to convert it into the approximated control variable model. The control variable model of Expression 4 includes the multiplication term (non-linear term) of the control variable $[X_v]$ and the amount of feedback adjustment (manipulated variable) $q_{B,\,FB}$ and the dynamic characteristics of the control system are described in a complicated relation. The approximation conversion unit 330 linearly approximates the non-linear term in the control variable model of Expression 4 in the vicinity of the operating point to convert it into the approximated control variable model of Expression 7.

$$\frac{d[X_v]}{dt} = \mu[X_v] - \frac{q_{B,FB}}{V}\overline{X_v} \tag{7}$$

In Expression 7, $X_V^-$ (wherein, $X_V^-$ shows $X_V$ with an over bar in Expression 7) represents the viable cell density approximated in the vicinity of the operating point and may be, for example, the same value as the target value of the process value of the viable cell density. Note that, in the actual cell culture process, $X_V^-$ can be regarded as the viable cell density of the bleed stream (the viable cell density in the cell culture liquid being discharged), because $X_V^-$ can be taken as a gain to multiply the bleed flow rate. Accordingly, the approximated control variable model of Expression 7 represents the steady state of the viable cell density control in an approximated manner. Note that, $[X_v]$ is the viable cell density in the culture tank 10 to be managed, and thus it is different from $X_V^-$.

In step S 15, the derivation unit 340 derives a closed-loop transfer function in the vicinity of the operating point based on the approximated control variable model and a control function that is used in control by the controller 13. The derivation unit 340 derives the closed-loop transfer function to which the feedback control law is applied. The closed-loop transfer function may represent a mathematical model of the entire control loop 15 which describes an input-output characteristic between at least one of the target value of the process value or an external input such as the disturbance and the control variable (output).

The derivation unit 340 may prestore the control function and acquire the information of the controller 13 through the acquisition unit 300 to use the control function that corresponds to a control law of the controller 13. In addition, the derivation unit 340 may acquire the control function from the operator 400 through the acquisition unit 300. For example, if the controller 13 is a PID controller, which is widely used in the cell culture process industry including the cell culture process, the manipulated variable is calculated by a control function that corresponds to the PID control scheme (PID control law). In this case, the derivation unit 340 may use the control function of Expression 8, as an example.

$$u(t) = K_p \cdot \left( e(t) + \frac{1}{T_I}\int e(t)dt + T_D\frac{de(t)}{dt} \right) \tag{8}$$

In Expression 8, u(t) represents the manipulated variable (MV), and e(t) represents a deviation (a difference between the process value (PV) and the target value (SV)). In addition, $K_P$, $T_I$, and $T_D$ are setting parameters, and $K_P$ represents a proportional gain, $T_I$ represents an integral time, and $T_D$ represents a derivative time. The adjustments of these PID parameters are very important in the setup operation of the controller 13 of the cell culture system 1 because they have a large influence on the control performance.

The derivation unit 340 can derive Expression 9 that represents the input-output relation between the PV (output) and the external signal (input), by combining the approximated control variable model and the control function of Expression 8 corresponding to the PID control scheme.

$$y = W_r(\boldsymbol{\theta_C}, \boldsymbol{\theta_{PM}}) \cdot r + W_d(\boldsymbol{\theta_C}, \boldsymbol{\theta_{PM}}) \cdot d \tag{9}$$

In Expression 9, y represents a process value (PV), r represents a target value (SV), and d represents a disturbance. $\theta_C$

represents a vector that aggregates the setting parameters and is described as $\theta_C = [K_P, T_I, T_D]$ when a control system of the PID controller is designed. Similarly, $\theta_{PM}$ is a vector that aggregates the parameters of the approximated control variable model. In addition, W, is a function that represents an input-output relation between the target value r and the control variable y, which is the process value, (target value response transfer function) and $W_d$ is a function that represents an input-output relation between the disturbance d and the control variable y (disturbance response transfer function), each being represented as a function of $\theta_C$ and $\theta_{PM}$.

As an example, the derivation unit 340 derives a closed-loop transfer function for the case in which the Integral-Proportional Derivative control law (I-PD control law) is applied to the bleeding operation of the viable cell density control system. Note that, in the I-PD control law, an arithmetic operation of the manipulated variable in response to a sudden change of the target value is more stable than in the standard PID control law, as represented in Expression 10.

$$u(t) = K_P \cdot \left( y(t) + \frac{1}{T_I} \int \{y(t) - r(t)\}dt + T_D \frac{dy(t)}{dt} \right) \tag{10}$$

In Expression 10, u(t) represents a manipulated variable, and in the viable cell density control system according to the bleeding operation, the amount of feedback adjustment $q_{B,\,FB}$ of the bleed flow rate corresponds to the u(t). Accordingly, the derivation unit 340 can derive the closed-loop transfer function by substituting Expression 10 into the approximated control variable model of the viable cell density control system as represented in Expression 7.

The derivation unit 340 converts the approximated control variable model of Expression 7 and an arithmetic expression (the controller 13 model) of Expression 10 into Expression 11 and Expression 12, respectively, to derive the closed-loop transfer function. In the present example, the differential and integral representations are replaced by algebraic descriptions by Laplace transform.

$$(s - \mu)y_{X_v} = -\frac{\overline{X_v}}{V} q_{B,FB} \tag{11}$$

$$q_{B,FB} = \left( K_P + \frac{K_P}{T_I s} + K_P T_D s \right) y_{X_v} - \frac{K_P}{T_I s} \cdot r_{X_v} \tag{12}$$

In Expression 11 and Expression 12, s represents a Laplace operator (corresponding to a differential operator in a time domain). In addition, $y_{X_v}$, $r_{X_v}$ represent a process value and a target value, respectively, of the viable cell density control.

The derivation unit 340 can derive the closed-loop transfer function (a target value response transfer function) $W_{X_v}$ of the viable cell density control in the vicinity of the operating point as shown in Expression 14, by substituting Expression 12 into Expression 11.

$$y_{X_v} = W_{X_v}(\boldsymbol{\theta_C}, \boldsymbol{\theta_{PM}}) \cdot r_{X_v} \tag{13}$$

$$W_{X_v}(\boldsymbol{\theta_C}, \boldsymbol{\theta_{PM}}) = \frac{1}{1 + \left( T_I - \frac{T_I}{K'_P}\mu \right)s + \left( \frac{T_I}{K'_P} + T_I T_D \right)s^2} \tag{14}$$

$$K'_P = \frac{\overline{X_v}}{V} \cdot K_P \tag{15}$$

$$\boldsymbol{\theta_C} = [K_P, T_I, T_D] \tag{16}$$

$$\boldsymbol{\theta_{PM}} = [\mu, \overline{X_v}, V] \tag{17}$$

In step S16, the control-target-function setting unit 350 sets the control target function according to the control target acquired by the acquisition unit 300. The control-target-function setting unit 350 may set the control target function in a binomial coefficient standard form, a Butterworth standard form, or an ITAE (Integral of Time multiplied by Absolute Error) least standard form. Because the control-target-function setting unit 350 takes a trade-off design principle in which the controller 13 is adjusted with a focus on one control characteristic and then specifications of other control characteristics are determined dependently, the control target function may be set in such a way that one control target acquired by the acquisition unit 300 is the control characteristic desired to be obtained preferentially in the cell culture process. For example, in the case of the viable cell density control in the perfusion culture, an adjustment of the setting parameters to follow the target value is studied in terms of the closed-loop stabilization for the cell growth or an improvement in the followability and responsiveness relative to the sequential review of the conditions (target value change). In this case, the setting parameters may be calculated using the target value response transfer function $W_r$.

The control-target-function setting unit 350 may prestore one or more control target functions each associated with a control target and set the control target function, among the stored control target functions, according to the control target acquired by the acquisition unit 300. In addition, the control-target-function setting unit 350 may receive, through the acquisition unit 300, a structure of the control target function and a designation of parameters input by the operator 400 and set the control target function that shows ideal performance for the control target according to the received structure of the control target function and the parameters. The control-target-function setting unit 350 may set the closed-loop transfer function as the control target function.

As an example, the control-target-function setting unit 350 sets the control target function $R^r_M$ related to the target value response characteristic (for example, an achievement target of the settling time, or the like) described in Non-Patent Document 2, as the binomial coefficient standard form of Expression 18.

$$R^r_M(\boldsymbol{\theta_{RM}}) = \frac{1}{(\tau s + 1)^n} e^{-L_M s} \tag{18}$$

$$\tau = \frac{T_{99}}{4.4\, n^{0.6}} \tag{19}$$

In Expression 18 and Expression 19, $T_{99}$ represents the achievement target of time for the process value to respond 99% (which corresponds to the settling time except the process dead time) relative to a step-wise change of the target value, $L_M$ represents an estimated process dead time, and n represents an order of the control target function. In addition, $\theta_{RM}$ represents a vector that aggregates the parameters of the control target function, and in the present example, $\theta_{RM} = [T_{99}, L_M, n]$.

The control-target-function setting unit 350 may set the control target function in a structurally similar manner to the closed-loop transfer function derived in step S15. The control-target-function setting unit 350 may set the control target function shown in Expression 20 so that it is similar to the structure of the closed-loop transfer function of Expression 14 derived in step S15.

$$R^r_M(\boldsymbol{\theta_{RM}}) = \frac{1}{(\tau s + 1)^2}$$
$$= \frac{1}{1 + 2\tau s + \tau^2 s^2} \tag{20}$$

Expression 20 is the control target function obtained by setting the parameters $L_M$ and n in the control target function shown in Expression 18 as $L_M = 0$, $n = 2$. In addition, the control-target-function setting unit 350 may determine the parameter $\tau$ of the control target function in Expression 20 by acquiring, through the acquisition unit 300, the designation by the operator 400 to $T_{99}$ (the settling time) in Expression 19.

In step S17, the setting-parameter generation unit 360 generates the setting parameters so that the closed-loop transfer function derived by the derivation unit 340 fits the control target function. The setting-parameter generation unit 360 may generate a conditional expression to cause the closed-loop transfer function to fit the control target function by performing a fit operation of the closed-loop transfer function and the control target function.

For example, the setting-parameter generation unit 360 may transform the closed-loop transfer function derived by the derivation unit 340 into a mathematical expression similar to the control target function and determine the relation between the coefficients (as represented by the function of $\theta_C$ and $\theta_{PM}$ shown in Expression 14) in the closed-loop transfer function and the coefficients in the control target function. As an example, the setting-parameter generation unit 360 is shown below as focusing on the closed-loop transfer function in Expression 9 and the control target function in Expression 18. First, for easier comparison between Expression 9 and Expression 18, the setting-parameter generation unit 360 may transform them each into a denominator series expression or the like as in Expression 21 and Expression 22.

$$W_r(\boldsymbol{\theta_C}, \boldsymbol{\theta_{PM}}) = \frac{1}{1 + c_1 s + \cdots c_n s^n + \cdots} \tag{21}$$

$$R_M^r(\boldsymbol{\theta_{RM}}) = \frac{1}{1 + \gamma_1 s + \cdots \gamma_n s^n + \cdots} \tag{22}$$

In Expression 21 and Expression 22, $c_n$ and $\gamma_n$ are the coefficient in the closed-loop transfer function and the coefficient in the control target function, respectively, derived by the denominator series expression, while $c_n$ is represented as the function of $\theta_C$ and $\theta_{PM}$ and $\gamma_n$ is represented as the function of $\theta_{RM}$.

From Expression 21 and Expression 22, the setting-parameter generation unit 360 can derive a simultaneous equation of $c_i = \gamma_i$ to be solved as the necessary condition for the closed-loop transfer function representing the dynamic characteristic of the control loop 15 to satisfy the control target function. As an example, the setting-parameter generation unit 360 can derive Expression 23 and Expression 24 because the closed-loop transfer function of Expression 14 already has a similar structure of mathematical expression to the control target function of Expression 20 as the conditional expression to fit the control target function.

$$2\tau = T_I - \frac{T_I}{K_P'} \mu \tag{23}$$

$$\tau^2 = \frac{T_I}{K_P'} + T_I T_D \tag{24}$$

Here, $K_P'$ in Expression 23 and Expression 24 is represented in Expression 15.

The setting-parameter generation unit 360 may generate the setting parameters from the conditional expressions obtained. The setting-parameter generation unit 360 may combine the conditional expressions obtained and derive an adjustment law for each setting parameter by uniquely representing the relation between the setting parameter $\theta_C$ and the parameter $\theta_{PM}$ of the approximated control variable model and the parameter $\theta_{RM}$ of the control target function. Thus, the adjustment law of the setting parameter to satisfy the control target function can be derived.

Depending on the closed-loop transfer function representing the dynamic characteristics of the control loop 15 and the control target function, more conditional expressions than the number of the setting parameters of the controller 13 may be obtained and the setting parameters are differently represented depending on the combination of the conditional expressions. In this case, the setting-parameter generation unit 360 may perform partial model matching to compare the coefficients in the same number as the number of the setting parameters from a low-order term (a term with a low order of s) of the control target function. For example, if the setting-parameter generation unit 360 performs parameter tuning using Expression 21 and Expression 22 on the control function (PID control law) represented in Expression 8, the setting parameters (PID parameters) are three ($K_P$, $T_I$, $T_D$), and thus the adjustment law of the setting parameter may be derived from the three conditional expressions of $c_1 = \gamma_1$, $c_2 = \gamma_2$, and $c_3 = \gamma_3$. The setting parameter $\theta_C$ can be represented as the function of the parameter $\theta_{PM}$ of the approximated control variable model and the parameter $\theta_{RM}$ of the control target function, as in Expression 25.

$$\boldsymbol{\theta_C} = f_{Control}(\boldsymbol{\theta_{PM}}, \boldsymbol{\theta_{RM}}) \tag{25}$$

In Expression 25, $f_{Control}$ is a function which indicates that the setting parameter satisfying the control target is represented by a combination of the parameter (the achievement target of the control characteristic) of the control target function and the parameter (the characteristic value of the cell culture process) of the approximated control variable model. Expression 25 indicates that the setting parameters can be tuned based on the characteristics of the cell culture process and the implementation requirements of the control, without a need of trial-and-error adjustment in establishing the setting parameters. The setting-parameter generation unit 360 can generate the setting parameter according to the derived adjustment law.

As an example, the derivation of the adjustment law is exemplified below using the conditional expressions of Expression 23 and Expression 24. In the present example, there are three setting parameters (proportional gain, integral time, derivative time) to be adjusted while only two conditional expressions are provided. In such a case, the setting-parameter generation unit 360 can derive the adjustment law by omitting the derivative element of the controller 13 because a unique solution satisfying the control target function is derived by the two setting parameters (there is the degree of freedom in the adjustment of the setting parameters). That is, for the target-value-following characteristic in the viable cell density control, the desired performance can be obtained even by the I-PD control law with the derivative time $T_D$ set to 0 (Integral-Proportional control law, I-P control law), although this is limited to the case in which Expression 20 is given as the achievement target of the closed-loop transfer function. Accordingly, Expression 26 obtained by setting $T_D = 0$ in Expression 24 can be handled as a new conditional expression.

$$\tau^2 = \frac{T_I}{K'_P} \tag{26}$$

The setting-parameter generation unit 360 can uniquely derive, as in Expression 27, the proportional gain $K_P$ and the integral time $T_I$ that satisfy the control target function in Expression 20 by organizing Expression 23, Expression 26, and Expression 16.

$$K'_P = \frac{2 + \mu\tau}{\tau} \tag{27}$$

$$K_P = \frac{V}{\overline{X_v}} \frac{2 + \mu\tau}{\tau} \tag{28}$$

$$T_I = (2 + \mu\tau)\tau \tag{29}$$

According to the above-described example of the mathematical expansion, it can be observed that the adjustment law of each setting parameter obtained in the present invention is described, as represented in Expression 25, by the function of the parameter $\theta_{RM} = [T_{99}, L_M, n]$ of the control target function representing the achievement target of the control characteristic and the parameter $\theta_{PM} = [\mu, X_V^-, V]$ of the approximated control variable model. The setting-parameter generation unit 360 can generate the setting parameter according to the adjustment law derived in this manner. The setting-parameter generation unit 360 may use the adjustment law to generate the setting parameter according to the operating condition such as the target value of the process value in the cell culture process.

The output unit 370 may output the setting parameter generated by the setting-parameter generation unit 360 to the controller 13 to cause the setting parameter to be set in the controller 13. Thus, the controller 13 performs the feedback control on the process value of the cell culture process according to the set setting parameter.

In step S18, the updating unit 380 may determine whether to update the setting parameter based on the operating condition of the cell culture process. The updating unit 380 may acquire, from the operator 400 or the controller 13 regularly or in real time, the operating condition of the cell culture process controlled by the controller 13 using the setting parameter generated in step S17 by the setting-parameter generation unit 360. The updating unit 380 may acquire, as the operating condition of the cell culture process, at least one of the process value of the cell culture process or the target value of the process value. The updating unit 380 may determine to update the setting parameter if the operating condition of the cell culture process exceeds a predetermined threshold or if the change of the operating condition from the time of generation of the setting parameter being used in the control of the cell culture process (for example, the difference between the operating condition at the time of generation of the setting parameter and the currently acquired operating condition) exceeds a predetermined threshold (as an example, $\alpha$). The updating unit 380 may determine to update the setting parameter according to an updating instruction from the operator 400.

When determining to update the setting parameter (Y in step S18 of Fig. 4), the updating unit 380 may request the setting-parameter generation unit 360 to update the setting parameter. In response to the request, the setting-parameter generation unit 360 may generate a new setting parameter using the operating condition currently acquired and cause the controller 13 to set it. As an example, the setting-parameter generation unit 360 may generate the new setting parameter by updating the parameter of the control variable model (or the approximated control variable model) using the specific growth rate $\mu$ in the currently acquired operating condition. The specific growth rate $\mu$ is often handled as a complicated function having time variability, and thus it is preferable that the update can be adaptively performed using the real-time process value.

When determining not to update the setting parameter (N in step S18 of Fig. 4), the updating unit 380 may end the operation of the setting parameter generation of the setting apparatus 14. The updating unit 380 may acquire new operating conditions during the operation of the cell culture process and repeat step S18 to determine whether to update the setting parameters. The updating unit 380 can update and adjust the setting parameter adaptively according to the operating situation at times because the setting parameter and the parameter of the approximated control variable model are derived with being associated with each other, as in Expression 25. The controller 13 can be operated so as to satisfy the desired control target throughout the entire culture section even if the dynamic characteristics of the control loop 15 changes according to the culture situation.

According to the present embodiment, the setting apparatus 14 can precisely set up the setting parameters in conformity with the mechanism of the cell culture process and thus save the effort of trial-and-error adjustment of the controller setting in the culture system on a trial run or the like, allowing a massive reduction in the amount of man-hour and the cost required to search for the setting parameters that satisfy the management range of the cell culture process. In addition, the setting parameters suitable for the operating situation at times can be updated promptly through the derived adjustment law even if the operating conditions (such as the specific growth rate, the perfusion rate (a ratio of the charged amount of the medium relative to the volume of the culture liquid per day), or the like) of the cell culture process temporally change.

Note that, for example, when the acquisition unit 300 newly acquires at least one of a control variable or a control target related to a different control system in the culture control process, the setting apparatus 14 may perform the steps S11 to S18 to generate new setting parameters.

In addition, when a plurality of control variables are acquired, the setting apparatus 14 may generate setting parameters for a plurality of control loops 15 in one flow of the setting parameter generation. If a multivariable interference is observed in a focused control loop 15, design requirements of each loop can be coordinated more easily by studying the specifications of the control system collectively rather than engineering them separately.

Note that, although the cell culture system 1 is described as performing the perfusion culture, it may perform the culture by other methods, such as a fed-batch culture or a continuous culture. In addition, the setting apparatus 14 may not include the adjustment unit 320, and in this case, the setting apparatus 14 may not perform the setting of the amount of feedforward adjustment but generate a setting parameter for a simple-feedback control system. In addition, if the control variable model is not non-linear, the setting apparatus 14 may not include the approximation conversion unit 330.

In addition, various embodiments of the present invention may be described with reference to flowcharts and block diagrams, wherein the block may serve as (1) a stage in a process in which an operation is performed, or (2) a section of an apparatus having a role of performing an operation. Certain stages and sections may be implemented by dedicated circuit, programmable circuit supplied together with computer readable instructions stored on computer readable media, and/or processors supplied together with computer readable instructions stored on computer readable media. Dedicated circuit may include digital and/or analog hardware circuits, and may include integrated circuits (IC) and/or discrete circuits. The programmable circuit may include a reconfigurable hardware circuit including logical AND, logical OR, logical XOR, logical NAND, logical NOR, and other logical operations, a memory element or the like such as a flip-flop, a register, a field programmable gate array (FPGA) and a programmable logic array (PLA), or the like.

A computer readable medium may include any tangible device that can store instructions to be executed by a suitable device, and as a result, the computer readable medium having instructions stored thereon includes a product including instructions that can be executed in order to create means for executing operations designated in the flowcharts or block diagrams. Examples of the computer readable medium may include an electronic storage medium, a magnetic storage medium, an optical storage medium, an electromagnetic storage medium, a semiconductor storage medium, and the like. More specific examples of the computer readable medium may include a floppy (registered trademark) disk, a diskette, a hard disk, a random access memory (RAM), a read-only memory (ROM), an erasable programmable read-only memory (EPROM or flash memory), an electrically erasable programmable read-only memory (EEPROM), a static random access memory (SRAM), a compact disc read-only memory (CD-ROM), a digital versatile disk (DVD), a Blu-ray (registered trademark) disk, a memory stick, an integrated circuit card, or the like.

The computer readable instruction may include: an assembler instruction, an instruction-set-architecture (ISA) instruction; a machine instruction; a machine dependent instruction; a microcode; a firmware instruction; state-setting data; or either a source code or an object code described in any combination of one or more programming languages, including an object oriented programming language such as Smalltalk (registered trademark), JAVA (registered trademark), C++, or the like, and a conventional procedural programming language such as a "C" programming language or a similar programming language.

Computer readable instructions may be provided to a processor of a general-purpose computer, special purpose computer, or other programmable processing apparatuses, or to programmable circuit, locally or via a local area network (LAN), wide area network (WAN) such as the Internet, or the like, to execute the computer readable instructions to create means for performing operations specified in the flowcharts or block diagrams. An example of the processor includes a computer processor, processing unit, microprocessor, digital signal processor, controller, microcontroller, or the like.

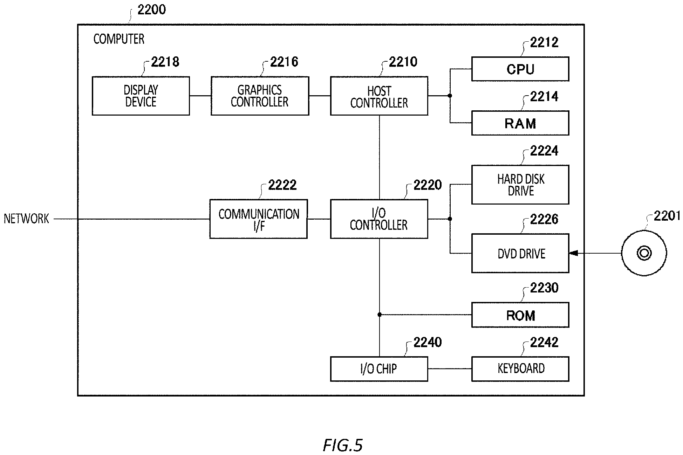

Fig. 5 shows an example of a computer 2200 in which a plurality of aspects of the present invention may be entirely or partially embodied. A program installed in the computer 2200 can cause the computer 2200 to function as an operation associated with the apparatuses according to the embodiments of the present invention or as one or more sections of the apparatuses, or can cause the operation or one or more sections to be executed, and/or can cause the computer 2200 to execute a process according to the embodiments of the present invention or a step of the process. Such programs may be executed by a CPU 2212 to cause the computer 2200 to perform specific operations associated with some or all of the blocks in the flowcharts and block diagrams described in the present specification.

The computer 2200 according to the present embodiment includes the CPU 2212, an RAM 2214, a graphic controller 2216, and a display apparatus 2218, which are interconnected by a host controller 2210. The computer 2200 also includes input/output units such as a communication interface 2222, a hard disk drive 2224, a DVD-ROM drive 2226, and an IC card drive, which are connected to the host controller 2210 via an input/output controller 2220. The computer also includes legacy input/output units such as an ROM 2230 and a keyboard 2242, which are connected to the input/output controller 2220 via an input/output chip 2240.

The CPU 2212 operates according to programs stored in the ROM 2230 and the RAM 2214, thereby controlling each unit. The graphic controller 2216 acquires image data generated by the CPU 2212 in a frame buffer or the like provided in the RAM 2214 or in itself, such that the image data is displayed on the display apparatus 2218.

The communication interface 2222 communicates with other electronic devices via a network. The hard disk drive 2224 stores programs and data used by the CPU 2212 in the computer 2200. The DVD-ROM drive 2226 reads a program or data from a DVD-ROM 2201 and provides the program or data to the hard disk drive 2224 via the RAM 2214. The IC card drive reads the programs and the data from the IC card, and/or writes the programs and the data to the IC card.

The ROM 2230 stores therein boot programs and the like executed by the computer 2200 at the time of activation, and/or programs that depend on the hardware of the computer 2200. The input/output chip 2240 may also connect various input/output units to the input/output controller 2220 via a parallel port, a serial port, a keyboard port, a mouse port, or the like.

Programs are provided by a computer readable medium such as the DVD-ROM 2201 or the IC card. The programs are read from the computer readable medium, are installed in the hard disk drive 2224, the RAM 2214, or the ROM 2230 which is also an example of the computer readable medium, and are executed by the CPU 2212. The information processing described in these programs is read by the computer 2200, and provides cooperation between the programs and the above-described various types of hardware resources. The apparatus or method may be configured by implementing operations or processing of information according to use of the computer 2200.

For example, in a case where communication is performed between the computer 2200 and an external device, the CPU 2212 may execute a communication program loaded in the RAM 2214 and instruct the communication interface 2222 to perform communication processing based on a processing written in the communication program. Under the control of the CPU 2212, the communication interface 2222 reads transmission data stored in a transmission buffer processing area provided in a recording medium such as the RAM 2214, the hard disk drive 2224, the DVD-ROM 2201, or the IC card, transmits the read transmission data to the network, or writes reception data received from the network in a reception buffer processing area or the like provided on the recording medium.

In addition, the CPU 2212 may cause the RAM 2214 to read all or a necessary part of a file or database stored in an external recording medium such as the hard disk drive 2224, the DVD-ROM drive 2226 (DVD-ROM 2201), the IC card, or the like, and may execute various types of processing on data on the RAM 2214. Then, the CPU 2212 writes the processed data back in the external recording medium.

Various types of information such as various types of programs, data, tables, and databases may be stored in a recording medium and subjected to information processing. The CPU 2212 may execute, on the data read from the RAM 2214, various types of processing including various types of operations, information processing, conditional judgement, conditional branching, unconditional branching, information retrieval/replacement, or the like described throughout the present disclosure and specified by instruction sequences of the programs, and writes the results back to the RAM 2214. In addition, the CPU 2212 may retrieve information in a file, a database, or the like in the recording medium. For example, when a plurality of entries, each having an attribute value of a first attribute associated with an attribute value of a second attribute, is stored in the recording medium, the CPU 2212 may retrieve, out of the plurality of entries, an entry with the attribute value of the first attribute specified that meets a condition, read the attribute value of the second attribute stored in said entry, and thereby acquiring the attribute value of the second attribute associated with the first attribute meeting a predetermined condition.

The programs or software modules described above may be stored in a computer-readable medium on or near the computer 2200. In addition, a recording medium such as a hard disk or an RAM provided in a server system connected to a dedicated communication network or the Internet can be used as a computer-readable medium, thereby providing a program to the computer 2200 via the network.

While the present invention has been described with the embodiments, the technical scope of the present invention is not limited to the above-described embodiments. It is apparent to persons skilled in the art that various alterations and improvements can be added to the above-described embodiments. It is also apparent from the description of the claims that the embodiments to which such alterations or improvements are made can also fall within the technical scope of the present invention.

It should be noted that the operations, procedures, steps, stages, and the like of each process performed by an apparatus, system, program, and method shown in the claims, the specification, or the drawings can be realized in any order as long as the order is not indicated by "prior to," "before," or the like and as long as the output from a previous process is not used in a later process. Even if the operation flow is explained using phrases such as "first" or "next" in the claims, specification, or drawings, it does not necessarily mean that the process must be performed in this order.

EXPLANATION OF REFERENCES

1: cell culture system;
10: culture tank;
11: actuator;
12: sensor;
13: controller;
14: setting apparatus;
15: control loop;
300: acquisition unit;
310: control-variable-model setting unit;
320: adjustment unit;
330: approximation conversion unit;
340: derivation unit;
350: control-target-function setting unit;
360: setting-parameter generation unit;
370: output unit;
380: updating unit;
101: sampling apparatus;
102: cell removal apparatus;
103: flow cell;
120: in-line sensor;
121: dielectric spectrometer
122: near-infrared/infrared/Raman spectrometer;
124: analyzer;
131: stirring apparatus
1210: electrode;
1220: light receiving and emitting sensor;
2200: computer;
2201: DVD-ROM;
2210: host controller;
2212: CPU;
2214: RAM
2216: graphics controller;
2218: display apparatus;
2220: input/output controller;
2222: communication interface;
2224: hard disk drive;
2226: DVD-ROM drive;
2230: ROM;
2240: input/output chip;
2242: keyboard.

**Claims**

1. An apparatus, comprising:

   an acquisition unit that acquires a control variable and a control target of a control object in a process controlled by a controller;
   a derivation unit that derives a closed-loop transfer function in a vicinity of an operating point of the process based on a control variable model that represents behavior of the control variable in the process and a control function used in control by the controller; and

a setting-parameter generation unit that generates a setting parameter for use in control by the controller so that the closed-loop transfer function fits a control target function that is set from the control target.

2. The apparatus according to claim 1, further comprising:

an approximation conversion unit that converts the control variable model into an approximated control variable model in which the control variable model is linearly approximated in a vicinity of an operating point of the process, wherein
the derivation unit derives a closed-loop transfer function in the vicinity of the operating point based on the approximated control variable model and the control function.

3. The apparatus according to claim 1, further comprising:
an adjustment unit that sets, to the control variable model, an amount of feedforward adjustment to offset at least one variable in the process.

4. The apparatus according to claim 1, wherein
the control function is a function that corresponds to a PID control scheme.

5. The apparatus according to claim 1, further comprising:
an updating unit that updates the setting parameter based on an operating condition of the process.

6. The apparatus according to claim 1, wherein
the setting-parameter generation unit generates the setting parameter using a partial model matching method so that the closed-loop transfer function fits the control target function.

7. The apparatus according to any one of claims 1 to 6, further comprising:
a control-target-function setting unit that sets the control target function based on the control target.

8. The apparatus according to claim 7, wherein
the control-target-function setting unit sets the control target function in a binomial coefficient standard form, a Butterworth standard form, or an ITAE (Integral of Time multiplied by Absolute Error) least standard form.

9. A method for controlling, by a controller, at least one of process values of a cell culture process using a setting parameter generated by the apparatus according to claim 1.

10. A controller that performs feedback control on at least one of process values of a cell culture process by a setting parameter generated by the apparatus according to claim 1.

11. A cell culture system, comprising:

a culture tank for culturing a cell or a microorganism in a cell culture process;
a sensor that measures a process value managed in the cell culture process;
an actuator that performs an operation to the culture tank; and
the controller according to claim 10 that controls the actuator according to a measurement result of the sensor.

12. A method, comprising:

acquiring a control variable and a control target of a control object in a process controlled by a controller;
deriving a closed-loop transfer function in a vicinity of an operating point of the process based on a control variable model that represents behavior of the control variable in the process and a control function used in control by the controller; and
generating a setting parameter for use in control by the controller so that the closed-loop transfer function fits a control target function that is derived from the control target.

13. A program that is executed by a computer and causes the computer to function as

an acquisition unit that acquires a control variable and a control target of a control object in a process controlled by a controller;

a derivation unit that derives a closed-loop transfer function in a vicinity of an operating point of the process based on a control variable model that represents behavior of the control variable in the process and a control function used in control by the controller; and

a setting-parameter generation unit that generates a setting parameter for use in control by the controller so that the closed-loop transfer function fits a control target function that is derived from the control target.

FIG.1

*FIG.2*

14

SETTING APPARATUS

400

OPERATOR

ACQUISITION
UNIT

300

310

CONTROL-VARIABLE-
MODEL SETTING UNIT

350

CONTROL-TARGET-
FUNCTION SETTING UNIT

380

UPDATING
UNIT

320

ADJUSTMENT
UNIT

330

APPROXIMATION
CONVERSION UNIT

340

DERIVATION
UNIT

360

SETTING-PARAMETER
GENERATION UNIT

370

OUTPUT
UNIT

CONTROLLER

13

FIG.3

```
                          ┌─────────────┐
                          │    START    │
                          └─────────────┘
                                 │
                                 ▼
        ┌──────────────────────────────────────────┐
  S11   │      ACQUIRE CONTROL VARIABLE             │
        │         AND CONTROL TARGET                │
        └──────────────────────────────────────────┘
                                 │
                                 ▼
        ┌──────────────────────────────────────────┐
  S12   │        SET CONTROL VARIABLE MODEL         │
        └──────────────────────────────────────────┘
                                 │
                                 ▼
        ┌──────────────────────────────────────────┐
  S13   │           SET AMOUNT OF                   │
        │      FEEDFORWARD ADJUSTMENT               │
        └──────────────────────────────────────────┘
                                 │
                                 ▼
        ┌──────────────────────────────────────────┐
  S14   │     CONVERT INTO APPROXIMATED             │
        │       CONTROL VARIABLE MODEL              │
        └──────────────────────────────────────────┘
                                 │
                                 ▼
        ┌──────────────────────────────────────────┐
  S15   │    DERIVE CLOSED-LOOP TRANSFER            │
        │      FUNCTION IN VICINITY OF              │
        │         OPERATING POINT                   │
        └──────────────────────────────────────────┘
                                 │
                                 ▼
        ┌──────────────────────────────────────────┐
  S16   │      SET CONTROL TARGET FUNCTION          │
        └──────────────────────────────────────────┘
                                 │ ◄──────────────┐
                                 ▼                │
        ┌──────────────────────────────────────────┐
  S17   │       GENERATE SETTING PARAMETER         │
        └──────────────────────────────────────────┘
                                 │                │
                                 ▼                │ Y
  S18   ◄──────────── UPDATE ? ───────────────────┘
                                 │ N
                                 ▼
                          ┌─────────────┐
                          │     END     │
                          └─────────────┘
```

*FIG.4*

FIG.5

Europäisches
Patentamt

European
Patent Office

Office européen
des brevets

# EUROPEAN SEARCH REPORT

**Application Number**

EP 24 20 4177

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2015 165344 A (TOSHIBA CORP) 17 September 2015 (2015-09-17) * the whole document * | 1-13 | INV. G05B13/04 |
| X | US 2003/195641 A1 (WOJSZNIS WILHELM K [US] ET AL) 16 October 2003 (2003-10-16) * the whole document * | 1,11-13 | |
| X | US 8 280 533 B2 (WOJSZNIS PETER [US]; BLEVINS TERRENCE LYNN [US] ET AL.) 2 October 2012 (2012-10-02) * the whole document * | 1,11-13 | |
| X | CN 101 578 584 A (UNIV STATE CLEVELAND [US]) 11 November 2009 (2009-11-11) * the whole document * | 1,11-13 | |

**TECHNICAL FIELDS SEARCHED (IPC)**

G05B
G06F

The present search report has been drawn up for all claims

| Place of search | Date of completion of the search | Examiner |
|---|---|---|
| The Hague | 14 February 2025 | José Luis Meseguer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 4177

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2025

| Patent document cited in search report | Publication date | Patent family member(s) | Publication date |
|---|---|---|---|
| JP 2015165344 A | 17-09-2015 | CN 106030423 A | 12-10-2016 |
| | | JP 6147687 B2 | 14-06-2017 |
| | | JP 2015165344 A | 17-09-2015 |
| | | WO 2015129677 A1 | 03-09-2015 |
| US 2003195641 A1 | 16-10-2003 | CN 1540461 A | 27-10-2004 |
| | | CN 101477333 A | 08-07-2009 |
| | | DE 102004019352 A1 | 10-11-2005 |
| | | GB 2400924 A | 27-10-2004 |
| | | HK 1065864 A1 | 04-03-2005 |
| | | JP 5015416 B2 | 29-08-2012 |
| | | JP 2004326781 A | 18-11-2004 |
| | | US 2003195641 A1 | 16-10-2003 |
| | | US 2007021850 A1 | 25-01-2007 |
| US 8280533 B2 | 02-10-2012 | CN 101930215 A | 29-12-2010 |
| | | CN 107526297 A | 29-12-2017 |
| | | EP 2267560 A1 | 29-12-2010 |
| | | GB 2471362 A | 29-12-2010 |
| | | JP 5697899 B2 | 08-04-2015 |
| | | JP 2011003186 A | 06-01-2011 |
| | | US 2009319060 A1 | 24-12-2009 |
| CN 101578584 A | 11-11-2009 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020252442 A
- US 2019153381
- WO 2020238918 A
- JP 2022537799 W
- JP 2019041656 A
- WO 2020039683 A
- WO 2021166824 A
- JP 2007202500 A
- JP 2018117567 A
- JP 2009510606 W
- JP SHO59045872 A
- JP 2012141791 A

**Non-patent literature cited in the description**

- **SEI MURAKAMI**. Bunkakai 2, Baiyo-koutei renzokuka no genjou to kadai (the current status and challenges of continuous culture process). *Seibutsu Kougakkaishi, Dai 97-kan, Dai 6-gou*
- **KANO, M.** ; **TASAKA, K.** ; **OGAWA, M.** ; **TAKINAMI, A.** ; **TAKAHASHI, S.** ; **YOSHII, S.** Extended fictitious reference iterative tuning and its application to chemical processes. *Proceedings of the 20114th International Symposium on Advanced Control of Industrial Processes*, 2011, 379-384